# EUROPEAN PATENT APPLICATION

(11) **EP 1 859 815 A1**
(43) Date of publication of application: **28.11.2007**
(21) Application number: 06010726.5
(22) Date of filing: 24.05.2006
(51) Int. Cl.: A61L 9/01, C01B 11/02

(54) **Method of reducing malodors**

(71) Applicant: THE PROCTER & GAMBLE COMPANY, Cincinnati, Ohio 45202 (US)
(72) Inventor: Scialla, Stefano, 00128 Rome (IT); Todini, Oreste, 1180 Brussels (BE)
(74) Representative: Canonici, Jean-Jacques

(57) **Abstract**

A method of reducing the formation of malodors in a bathroom. More specifically, it is provided a method of reducing the formation of malodors comprising the step of placing a chlorine dioxide generating sachet in selected locations of a bathroom.

## Description

### Technical field

The present invention relates to a method of reducing the formation of malodors in a bathroom. More specifically, the present invention is directed to a method of reducing the formation of malodors comprising the step of placing a chlorine dioxide generating sachet in selected locations of a bathroom.

### Background of the invention

Air freshener or deodorizing systems have long been sought by consumers, in both residential and commercial environments. In an attempt to meet the demand for air fresheners or deodorizers, numerous products have been developed and are presently available in the martketplace. In general, these prior art products are sold as solids, liquids, gel or aerosol sprays to provide the desired effect. Typically, these prior art products are used to eliminate, chemically change, or mask an existing odor. Products known in the art typically work by absorbing odorous molecules, dissolving or emulsifying such molecules, or killing bacteria that cause the offensive odor.

Among the variety of offensive odors and the variety of spaces where such malodors might be generated, confined spaces are particularly prone to develop unwanted odors. In the context of household applications, bathrooms are particularly subject to development of malodors.

Chlorine dioxide has long been recognized as useful for deodorizing a variety of malodours. Chlorine dioxide, which is gaseous at room temperature and atmospheric pressure, is known to be toxic to humans at relatively high concentrations and may be explosive at concentrations above 0.1 atmosphere. However, gaseous chlorine dioxide is generally considered safe for human contact at the low concentrations that are effective for deodorization. These problems have therefore considerably limited the use of chlorine dioxide to large commercial applications. Chlorine dioxide has, in particular, not achieved widespread use in household applications which involve contact with human.

Partial solutions to these drawbacks have been provided with for example in WO 02/23993 and US-A1-2004/0183050, which discloses methods of reducing odors comprising contacting a surface with a chlorine dioxide generating composition. Another solution is described in US-B2-6,676,850 which discloses a method of sanitizing and/or deodorizing a solid surface comprising exposing a surface with a composition capable of generating chlorine dioxide upon exposure to water vapor. The problem of providing long-term deodorizing or air freshening is particularly critical when trying to install a pleasant odor in areas or environments in which offensive odors continuously exist, such as in bathrooms.

It is therefore an objective of the present invention to provide a method of reducing the formation of a broad variety of malodors which are particularly prone to develop in a bathroom.

Advantageously, the method according to the present invention is capable of providing long-term and sustained malodors reduction or air freshening. Also the method of the present invention is able to provide malodor reduction or air freshening in a highly controlled manner without requiring any intervention of the user. A further advantage associated with the method according to the present invention is that it involves very simple and inexpensive material, and may be easily employed in both residential and commercial establishments for providing malodors reduction or air-freshening in bathrooms. It is still a further advantage that the method of the present invention is completely safe for use by humans.

Other advantages and more specific properties of the method according to the present invention will be clear after reading the following description of the invention.

### Summary of the invention

The present invention relates to a method of reducing the formation of malodors in a bathroom comprising the step of placing a moisture-permeable, water-insoluble sachet comprising a dry chlorine dioxide precursor and a dry chlorine dioxide activator in close proximity of a bathtub and/or shower present in the bathroom.

In another embodiment, the present invention is directed to a method of freshening air in a bathroom, the method comprising the step of placing a moisture-permeable, water-insoluble sachet comprising a dry chlorine dioxide precursor and a dry chlorine dioxide activator in close proximity of a bathtub and/or shower present in the bathroom.

The present invention further encompasses the use of a moisture-permeable, water-insoluble sachet comprising a dry chlorine dioxide precursor and a dry chlorine dioxide activator, for reducing the formation of malodors in a bathroom

### Detailed description of the invention

### SACHET

The method according to the present invention involves using a moisture-permeable, water-insoluble sachet. The sachet for use herein may be formed from any material well known in the art of functional sachets for being moisture-permeable and water-impervious. Typically, the sachet for use herein may be defined as being formed from a microporous membrane comprising one or more selected from the group consisting of woven or non-woven, synthetic or natural fibers integrally joined together, wood pulp, and plastic films or sheets. Preferably, the sachet for use in the present invention is formed from a polymeric plastic sheet comprising one selected from the group consisting of polyurethanes, polyethers, polyesters, polypropylenes, polystyrenes, and combinations thereof.
In an even more preferred embodiment, the sachet for use herein is made from materials commercially available under the tradenames TYVEK® and GORTEX®, most preferably from TYVEK®. Such particular materials enable water vapor to enter into the sachet and the resulting chlorine dioxide gas to be released from the sachet and enter the surrounding atmosphere, while said material being substantially impervious to water.

Suitable sachet may have any suitable configuration, form or dimension for accommodating the dry chlorine dioxide activator and the dry chlorine dioxide precursor. Suitable sachet for use in the present invention will easily be recognized by those skilled in the art. As a way of example, suitable sachets for use herein are described e.g. US-B1-6,294,108 on column 9, lines 53-63. In a preferred embodiment the sachet for use herein has a substantially rectangular shape, preferably a rectangular shape, although the present invention is not so limited. However, it will be easily apparent to those skilled in the art that, depending upon the particular aesthetic impression which is ultimately aimed at, other configurations and shapes of the sachet may be used.

According to an alternative embodiment of the present invention, the sachet for use herein may be incorporated within a suitable container which may in turn be provided with aesthetic features. Suitable containers for use herein will be easily recognized by those skilled in the art. Typically, such containers shall be provided with suitable openings as required for the released chlorine dioxide to diffuse through said container.
As a way of example, suitable containers include but are not limited to, a box, a bottle, a pouch, an envelope, a can, a tube, and a bag.

### DRY CHLORINE DIOXIDE PRECURSOR

According to the present invention, the moisture-permeable, water-insoluble sachet for use herein further comprises a dry chlorine dioxide precursor. Preferably, the dry chlorine dioxide precursor for use in the context of the present invention is a metal chlorite. Preferred metal chlorites are alkali metal chlorites, such as sodium chlorite and potassium chlorite. Also suitable for use herein are alkaline earth chlorites. Examples of such alkaline earth chlorites include but are not limited to barium chlorite, calcium chlorite, and magnesium chlorite. The most preferred metal chlorite for use in the present invention is sodium chlorite.

### DRY CHLORINE DIOXIDE ACTIVATOR

As indicated above, the moisture-permeable, water-insoluble sachet for use herein further comprises a dry chlorine dioxide activator. The dry chlorine dioxide activator for use herein is any material capable of reacting with said dry chlorine dioxide precursor to produce chlorine dioxide gas in presence of water vapor. In a very preferred embodiment, said dry chlorine dioxide precursor and said dry chlorine dioxide activator are capable of reacting with each other to produce chlorine dioxide gas in the presence of water vapor, but do not generate chlorine dioxide in the substantial absence of liquid water or water vapor.

Preferably, the dry chlorine dioxide activator for use in the context of the present invention is a dry solid hydrophilic material, preferably a dry solid inorganic hydrophilic material. Examples of suitable dry solid inorganic hydrophilic material for use in the context of the present invention include but are not limited to, synthetic zeolites, such as A, X, Y, and mordenite; natural zeolites such as chabazite and clinoptilolite; hydrous clays, such as bentonite, kaolin, attapulgite and halloysite; calcinated clays, such as metakaolin, spinel phase kaolin, calcinated bentonite, calcinated halloysite, and calcinated attapulgite; acidified synthetic zeolites, such as A, X, Y, and mordenite that have been contacted with one or more acidic solutions containing sulfuric acid, hydrochloric acid, nitric acid, or other acidic compound (e.g. calcium chloride) so that the pH of the resulting aqueous phase of the mixture is below 10.5; acidified natural zeolites such as chabazite and clinoptilolite; acidified clays, such as bentonite, kaolin, attapulgite and halloysite that have been contacted with one or more acidic solutions containing sulfuric acid, hydrochloric acid, nitric acid, or other acidic compound (e.g. lanthanum chloride) so that the pH of the resulting aqueous phase of the mixture is below 10.5; acidified calcinated clays, such as metakaolin, spinel phase kaolin, calcinated bentonite, calcinated halloysite, and calcinated attapulgite that have been contacted with one or more acidic solutions containing sulfuric acid, hydrochloric acid, nitric acid, or other acidic compound (e.g. acetic acid) so that the pH of the resulting aqueous phase of the mixture is below 10.5; salts, such as aluminium sulfate, magnesium sulfate, calcium carbonate, and particularly deliquescent acidic slats, such as calcium chloride, magnesium chloride, chloride and magnesium nitrate; solid acids, such as boric acid, tartaric acid and citric acid; organic acid anhydrides such as phthalic anhydride, maleic anhydride, succinic anhydride and glutaric anhydride; and mixtures thereof.

In a preferred embodiment of the invention, the dry chlorine dioxide activator for use herein is selected from the group consisting of calcinated clays, acidified synthetic zeolites, acidified natural zeolites, acidified calcinated clays, and mixtures thereof. More preferably, the dry chlorine dioxide activator for use in the present invention is selected from acidified calcinated clays.

In accordance with the present invention, the mixture of the dry chlorine dioxide precursor and the dry chlorine activator generates the chlorine dioxide gas in a sustained concentration of from 0.001 to 1000 ppm, preferably from 0.001 to 100 ppm, more preferably from 0.01 to 10 ppm, and most preferably from 0.01 to 0.1 ppm. The measurement of chlorine dioxide gas is made in the atmosphere into which the chlorine dioxide gas is generated. For example, if the generating mixture is exposed to water vapor in air, the concentration of chlorine dioxide gas in ppm will be measured based upon the total atmosphere including air and water vapor. Also, the generation of chlorine dioxide need not be at a constant rate. It is permissible to have a fluctuating rate so long as the chlorine dioxide gas concentration does not exceed, for a sustained period of time, the limits which are safe for human contact depending upon the particular environment where the chlorine dioxide generating sachet is being used.

The reaction of said dry chlorine activator and said dry chlorine dioxide precursor may last for a sustained period of time, i.e. the chlorine dioxide gas will be generated during a short period of time (e.g. several minutes) to a long period of time spanning several hours or weeks. The length of the sustained period of time will depend upon the relative amounts of the constituents in the mixture. In any case, during the course of the reaction, chlorine dioxide gas will be produced in a sustained concentration as defined above.

According to a preferred embodiment of the invention, the generation of chlorine dioxide gas within the specified range will vary depending on the relative humidity of the surrounding atmosphere, the ratio of the reactants, the diluent gas flow rate (e.g. air) through the treated space, and the ratio of the amount of chlorine dioxide gas releasing material to the volume of the treated space. Generally, the higher the relative humidity the higher rate of prod.uction of chlorine dioxide gas. The lower the flow of the diluent gas through the treated space, the higher the resultant chlorine dioxide gas concentration.
The amount of each of the dry chlorine dioxide precursor and said dry chlorine dioxide activator will depend on several factors, including but not limited to, the quantity of chlorine dioxide gas needed for as particular application, the basicity of the dry chlorine dioxide precursor and the acidity of the dry chlorine dioxide activator. In general, the weight ratio of the chlorine dioxide precursor and the dry chlorine dioxide activator is in the range of from 0.001 to 0.25:1.0. It is well within the capability of the skilled person to choose the proper ratio suitable for a particular application.

In accordance with a preferred embodiment of the present invention, said dry chlorine dioxide precursor and said dry chlorine dioxide activator are admixed so as to form a mixture. Such mixture may be formulated in several ways which will be easily recognized by those skilled in the art. The preferred method is to prepare in a dry atmosphere an intimate physical mixture of fine powders of both constituents having particle sizes preferably below 200 µm. Larger particles may be used and may achieve a slower rate of chlorine dioxide gas release in certain circumstances.

In a preferred execution of the present invention whereby a slow release rate of long duration is produced, the mixture comprises 5 wt% of dry chlorine dioxide precursor and 95% of dry chlorine dioxide activator. A preferred mixture for a shorter duration, higher rate of generation of chlorine dioxide is a mixture of 5 wt% of dry chlorine dioxide precursor, 10 wt% of dehydrating agent and the balance of dry chlorine dioxide activator.

It will be understood that for a given unit of the mixture represented by a unitary sachet, a sustained amount of chlorine dioxide gas will he produced. For some specific applications, it may be desirable to employ multiple units of the mixture to achieve the desired deodorizing effect.

According to the preferred embodiment wherein said dry chlorine activator and said dry chlorine dioxide precursor are not capable of reacting with each other to produce chlorine dioxide gas in the substantial absence of water, both reagents may be prepared in advance and stored for prolonged time under dry conditions without premature release of chlorine dioxide gas. In accordance with this preferred embodiment of the invention, it is allowed to achieve a precise control of the concentration, strength and rate of release of chlorine dioxide.
In the practice of the present invention, the relative humidity of the atmosphere to which the mixture is exposed during use can range from low humidity (e.g. 10% relative humidity) up to 100% relative humidity conditions. As above indicated, the amount of chlorine dioxide gas generated per given amount of the mixture will depend, in part, on the relative humidity of the surrounding atmosphere. In general, higher humidity will result in a higher concentration of chlorine dioxide gas.

### OPTIONAL FEATURES

The mixture formed in accordance with the present invention may optionally comprise at least one dehydrating agent which is primarily intended to absorb water to minimize or eliminate an initial brief duration production of chlorine dioxide due to residual water vapor present in the atmosphere when the mixture is packaged within the moisture permeable, water-insoluble sachet. Also, it has been surprisingly discovered that the combination of chlorine dioxide precursor and said dry chlorine dioxide activator together with a dehydrating agent contributes to obtain improved deodorizing effect.
The optional use of dehydrating agent to minimize chlorine dioxide gas production in the sachet during storage can ensure that the mixture will react for the longest period of time when exposed to water vapor under operating conditions. The presence of dehydrating agent may delay the desired onset of production of chlorine dioxide gas when the mixture is exposed to water vapor. Therefore, the length of time of the reaction is also dependent, in part, on how much water vapor is present in the atmosphere contained within the sachet.

Alternatively, said dehydrating agent may be kept separate from said moisture-permeable, water insoluble sachet. According to this alternative embodiment, the method according to the present invention involves the use of a dehydrating agent as a distinct element from the water-insoluble sachet.

Suitable dehydrating agents for use herein include but are not limited to, activated calcium chloride, activated calcium sulfate, activated zeolite X, activated zeolite A, activated bentonite clay, activated silica gel, activated attapulgite, and mixtures thereof. The term "activated' means that the particular material has been substantially dehydrated, for example, by heating at 300C for one hour. The total amount of dehydrating agent may vary depending on several factors, for example, the ambient humidity when the material is packaged into the suitable sachet, the water permeability of the sachet material and the desired shelf life of the product. Typically, the dehydrating agent is present in a total amount from 0.1 % to 25% by weight based o the total weight of the mixture

The method of the present invention may optionally, but preferably, involve the use of a perfume composition. It has been indeed discovered that the use of gaseous chlorine dioxide may be offending to the olfactory senses and may even be perceived by consumers as unacceptable because of its unpleasant odor. This unrecognized problem may detrimentally affect the overall deodorizing benefit provided by the method of the present invention.

As used herein the term "perfume" is used to indicate any odoriferous material that is subsequently released into the ambient air. The perfume will most often be liquid at ambient temperatures. A wide variety of chemicals are known for perfume uses, including materials such as aldehydes, ketones, and esters. More commonly, naturally occurring plant and animal oils and exudates comprising complex mixtures of various chemical components are known for use as perfumes. The perfumes herein can be relatively simple in their compositions or can comprise highly sophisticated complex mixtures of natural and synthetic chemical components, all chosen to provide any desired odor. Typical perfumes can comprise, for example, woody/earthy bases containing exotic materials such as sandalwood, civet and patchouli oil. The perfumes can be of a light floral fragrance, e.g. rose extract, violet extract, and lilac. The perfumes can also be formulated to provide desirable fruity odors, e.g. lime, lemon, and orange.
Likewise, the perfumes delivered in the compositions and articles for use in the method of the present invention may be selected for an aromatherapy effect, such as providing a relaxing or invigorating mood. As such, any material that exudes pleasant or otherwise desirable odors can be used as a perfume active in the method of the present invention. Perfume materials are described more fully in S. Arctander, Perfume Flavors and Chemicals. Vols. I and II. Aurthor, Montclair, N.J., and the Merck Index, 8th Edition, Merck & Co., Inc. Rahway, N.J.

According to the present invention, perfume composition for use herein may either be part of the moisture-permeabte, water-impervious sachet or alternatively may be kept separate from said sachet.
In the specific embodiment of the invention wherein the perfume composition is part of the sachet, the composition may be loaded onto the external surface of the sachet or onto the internal surface of the sachet. Suitable techniques for loading said perfume composition onto the sachet will be easily recognized by the skilled person. For example, perfume composition may be incorporated directly within the material used to form such sachet, or may applied onto the surface of said sachet by means of printing or spraying.
Alternatively, said perfume composition may be incorporated in the mixture together with said dry chlorine activator and said dry chlorine dioxide precursor, inside said sachet. In such specific embodiment of the invention, the perfume composition may be selected from any perfume composition commonly known as being compatible with oxidizing agents, and more particularly with oxidizing gases. Suitable perfume composition for use according to this particular embodiment of the invention may be easily determined by those skilled in the art of perfumery. As a way of example, suitable perfume compositions are described e.g. in US-B1-6,255,268 on column 6 line 10 to column 9 line 50. Other examples of suitable perfume compositions are disclosed in EP-B1-0622451 on column 3 lines 22-50.

According to a preferred execution however, the perfume composition for use in the context of the present invention is kept separate from said moisture-permeable, water-impervious sachet. In accordance with this specific embodiment, suitable perfume composition may be selected from any perfume composition commonly known in the art of perfumery including perfume composition know to be chemically incompatible with oxidizing gases. It will be understood that one of ordinary skill in the art, may easily recognize suitable perfume composition for use in the present invention. Typically, the perfume composition for use in accordance with this specific embodiment is a mixture of organic compounds admixed so that the combined odors of the individual components produce a pleasant or desired fragrance. Suitable perfume composition for use in accordance with the present embodiment may take any physical form including solid, particulate, gel, liquid, paste, tablet, bar, gas, and mixtures thereof. In a preferred embodiment, the perfume composition for use herein is in the form of liquid, solid, gel, adhesive gel, paste, tablet, and mixtures thereof.

In a preferred execution of the present invention, the perfume composition for use herein is a polymeric composition obtained hy combining a low melting point polyamide polymer with a polar thermoplastic elastomer and a perfume. Such polymeric compositions are described for example in US-A1-2006/0099168.
The term "low melting point polyamide polymers" includes all polyamides having a melting point below 130°C, preferably below 110°C, more preferably below 100°C. Typically and preferably, the low melting point polyamides for use in the present invention are solid at room temperature. Preferred polyamides are terminated polyamides, particularly preferred are ester terminated polyamides. Examples of these low melting point polyamides include those marketed by Arizona chemicals under the trade name of SYLVACLEAR®.
The term "polar thermoplastic elastomer" includes multiphase polymers that comprise "hard" and "soft" phases chemically bonded together in the polymer chain. The "hard" phase is solid at room temperature and flows upon heating. Examples include blocks of amide, ester and urethane groups. The "soft" phase is rubbery at room temperature.
Examples include polyether blocks such as poly(ethylene glycol), poly(propylene glycol) or poly(tetramethylene glycol). At room temperature, the presence of the "hard" phases in the polymer imparts strength and good mechanical properties. When the polymer is heated, these phases become liquid and the polymer melts, allowing for processing in the molten state. Upon recooling to room temperature, the phases solidify and the good mechanical properties are regained. A comprehensive definition of thermoplastic elastomers can be found in Vol 9 of the Kirk-Othmer Encyclopedia of Chemical Technology (4th Edition - Wiley- Interscience, 1996) - under the voice "Elastomers", subvoice "Thermoplastic Elastomers". Among these polymers those which are suitable for the present invention are those comprising at least one polar monomer. Polar monomers are those monomers which comprise at least a C-X linkage in the molecule wherein said C-X linkage is a polar linkage. Preferably X is an N, S, F, Cl or O atom. More preferably said polar linkage is part of a carbonyl group and, more preferably, of an ester group. Preferred polar monomers for the present invention are vinyl acetate, vinyl alcohol, methyl acrylate, ethyl acrylate, butyl acrylate, acrylic acid and salts formed therefrom, methacrylic acid and salts formed therefrom, maleic anhydride, glycidyl methacrylate and carbon monoxide. More preferably the hard phases preferably comprise blocks of amide, ester or urethane groups and the soft phases preferably comprise polyether blocks. Examples of these polar thermoplastic elastomers include thermoplastic polyurethanes, such as those produced under the trade names ESTANE® by Noveon, and PELLETHANE® by Dow Chemicals; thermoplastic polyesters, also known as polyether ester copolymers, such as those produced under the trade names HYTREL® by Dupont and ARNITEL® by DSM, and thermoplastic polyamides, also known as polyether amide copolymers, such as those produced under the trade name PEBAX® by Atofina.

According to a highly preferred execution of the present invention, the perfume composition for use herein is a polymeric composition obtained by combining an ester terminated polyamide, a thermoplastic polyether amide copolymer and a perfume, according to the method described in US-A1-2006/0099168.

Preferably, the perfume composition is provided with an aesthetically appealing shape or form. Depending upon the ultimately desired aesthetic effect, the perfume composition may alternatively be comprised within a distinct container which may in turn be provided with aesthetic features. Suitable containers for use herein will be easily recognized by those skilled in the art of perfumery. Typical containers include but are not limited to, a box, a bottle, a pouch, an envelope, a can, a tube, beads, flakes and bags.

Typically, such containers shall be provided with suitable openings as required for perfume composition to diffuse through said container. Such openings will also allow free circulation of air within said container.

According to the present invention, the Applicant has surprisingly discovered that by providing a method involving the use of a perfume composition, the potential user is not overcome by the unpleasant odor associated with chlorine dioxide gas. The present invention therefore provides the user with a more pleasant deodorizing experience since the method according to the invention does not only neutralize malodors which may be present in a bathroom but also delivers a desired fragrance unaffected by any other unwanted side malodors.

As a further optional feature, the water-insoluble sachet for use in the method of the invention may comprise a visual means indicating when the water-insoluble needs to be replaced. Suitable visual means for use herein may be any such visual means commonly known in the art of functional sachets. Typical visual means for use herein comprise but are not limited to badges or patches based upon colour-changing or degrading material technologies. Preferably, the visual means is selected to be a patch based upon colour-changing technology.

### METHOD OF REDUCING THE FORMATION OF MALODORS

The present invention is primarily directed to a method of reducing the formation of malodors in a bathroom comprising the step of placing a moisture-permeable, water-insoluble sachet comprising a dry chlorine dioxide precursor and a dry chlorine dioxide activator in close proximity of a bathtub and/or shower present in said bathroom.

By "close proximity", it is meant herein that the moisture-pemeable, water-insoluble sachet is placed at a distance not exceeding 3 meters, preferably not exceeding 2 meters, more preferably not exceeding 1 meter, in any directions from said bathtub and/or shower present in the bathroom.

The method of the present invention may be useful for reducing the formation af a broad variety of malodors which may particularly develop in bathrooms. Typical examples of such malodors include, but are not limited to mold and mildew odors, odors from soap scum and body soil residues, pipe malodors, smoke; cleaning or personal care products malodors and pet litters. More specifically, the method according to the present invention is particularly adapted for reducing mold and mildew odors.

In the context of the present invention, it has surprisingly discovered that by placing a moisture-permeable, water-insoluble sachet as above described in close proximity of a bathtub and/or shower, enhanced reduction of malodors is achieved when compared to the situation where such water-insoluble sachet is placed in any other locations within said bathroom. The present invention is based on the discovery that specific locations in a bathroom, which are characterized by a relative high humidity, are particularly prone to develop malodors. As above indicated, the amount of chlorine dioxide gas generated by the water-insoluble sachet for use herein will depend, for a large part, on the relative humidity of the surrounding atmosphere. More specifically, the higher humidity will result in a higher concentration of chlorine dioxide gas. In other words, the method according to the present invention permits a targeted, controlled and adaptable action towards reducing the formation of malodors in a bathroom.

According to the method of the invention, the moisture-permeable, water-insoluble sachet may be put down on a flat surface or may alternatively hang on a hook or similar holder. Preferably, the sachet is hanged up on a hook. According to this preferred execution of the invention, it is believed that improved water vapor circulation within said sachet is achieved and therefore enhanced reduction of the formation of malodors is consequently obtained. It is understood that the person skilled in the art as well as the potential user will easily determine the most suitable locations for placing the water-insoluble sachet, in order to achieve the desired deodorizing benefit.
Also, depending upon the intensity of the malodors to be reduced, the user may find useful to place one or more units of said moisture-permeable, water-insoluble sachet in the bathroom to be treated.
Accordingly, the articles for use in the context of the present invention may optionally comprise specific instructions which may help the user to select the number of sachets to use depending upon the bathroom and the specific malodors to be treated and to choose specific suitable locations for said sachet within said bathroom.

Such set of usage instructions for selecting and dosing the sachet and/or the optional articles for use in the present invention, may be provided in any suitable locations such as in a pamphlet, a computer screen, a printed ticket, a kiosk, a sign, a product container, an advertisement, a product display, an Internet website, a video, and a combination thereof. Preferably the set of usage instructions are provided on the sachet container, a product display, or a combination thereof, as these locations are easy to reference. More preferably, the set of usage instructions are provided on the sachet container, as the set of usage instructions is thus unlikely to become lost and/or separated from the sachet when it is needed. Alternatively, the set of usage instructions are provided on any of the optional articles for use herein. Without intending to be limited by theory, it is believed that such set of usage instructions may significantly reduce misuse and/or inappropriate use by the consumer of the sachet and/or the different optional articles for use in the present invention. Also, the set of usage instruction may help the user achieving an improved deodorizing effect.

Incidentally, and according to another embodiment of the present invention, it is provided a method of reducing humidity in a bathroom due to the water-vapor absorbing properties of the water-insoluble sachet for use in the present invention.
In accordance with the present invention wherein said dry chlorine activator and said dry chlorine dioxide precursor are only able to produce chlorine dioxide gas when exposed to moisture vapor in ambient atmosphere, the method according to the invention provides the additional benefit that the treated bathrooms are kept fresher since the ambient humidity is absorbed by the water-insoluble sachet for use in the present invention. Therefore, the method according to the present invention may incidentally operate as a method for dehumidifying a bathroom. In that sense, the method according to the present invention may be particularly beneficial to persons suffering from rheumatism when exposed to humidity.

### USE OF THE SACHET FOR REDUCING FORMATION OF MALODORS

The present invention is further directed to the use of a moisture-permeable, water-insoluble sachet comprising a dry chlorine dioxide precursor and a dry chlorine dioxide activator, for reducing the formation of malodors in a bathroom.

### Examples

### Example 1:

A moisture-permeable, water-impervious sachet (about 50 grams) available from Engelhard Corporation under the tradename Aseptrol® is hanged in a regular bathroom at 1 meter from the bathtub. A perfume composition in solid form (about 200 grams) is obtained by charging 70 parts of Lavender natural extract into a vessel (sealed or under reflux) together with 10 parts of sucrose acetate isobutyrate (SAIB from Eastman Chemical) as plasticizer and mixed at room temperature. The temperature is then elevated to 80°C. 10 parts of Pebax® 2533 (from Total Fina) as polar thermoplastic elastomer and 10 parts of low melting point polyamide Sylvaclear® AF 1900 from Arizona Chemical are charged into the vessel and stirred till complete dissolution. The composition is then let to cool down and solidify at room temperature. The obtained perfume composition is placed on a flat surface at about 1 meter from said water-impervious sachet. The formation of malodors, for instance mold and mildew malodors, are reduced for about 1 month and no chlorine dioxide odor is noticeable during that period. Also, ambient air is perceived as fresher due to the reduction of persistent humidity inside the treated bathroom.

### Example 2:

A moisture-permeable, water-insoluble sachet (about 100 grams) and a perfume composition (about 200 grams) as described in example 1 are placed in distinct cardboard-made containers provided with openings. Both containers are hanged to the roof of a regular basement at about 1 meter distance from each other. The formation of malodors, for instance mold and mildew malodors, are reduced for about 1 month and no chlorine dioxide odor is noticeable during that period. Also, ambient air is perceived as fresher due to the reduction of persistent humidity inside the treated basement.

## Claims

1. A method of reducing the formation of malodors in a bathroom comprising the step of placing a moisture-pemeable, water-insoluble sachet comprising a dry chlorine dioxide precursor and a dry chlorine dioxide activator in close proximity of a bathtub and/or shower present in said bathroom.

2. A method according to claim 1 wherein said dry chlorine dioxide precursor is selected from metal chlorites, preferably alkali metal chlorites.

3. A method according to claim 2 wherein said dry chlorine dioxide precursor is sodium chlorite.

4. A method according to any preceding claims wherein said dry chlorine dioxide activator is selected from the group consisting of calcinated clays, acidified synthetic zeolites, acidified natural zeolites, acidified calcinated clays, and mixtures thereof.

5. A method according to claim 4 wherein said dry chlorine dioxide activator is selected from acidified calcinated clays.

6. A method according to any preceding claims wherein the weight ratio of said dry chlorine dioxide precursor and said dry chlorine dioxide activator is in the range from 0.001 to 0.25:1.0.

7. A method according to any preceding claims wherein said dry chlorine dioxide precursor and a dry chlorine dioxide activator are admixed so as to form a mixture.

8. A method according to claim 7 wherein said mixture comprises 5 wt% of said dry chlorine dioxide precursor and 95 wt% of said dry chlorine dioxide activator.

9. A method according to claim 7 wherein said mixture further comprises a dehydrating agent.

10. A method according to claims 9-10 wherein said mixture comprises from 0.1 wt% to 25 wt% of said dehydrating agent.

11. A method according to claims 1-8 which further comprises a dehydrating agent as a distinct element.

12. A method according to any preceding claims wherein said dehydrating agent is selected from the group consisting of activated calcium chloride, activated calcium sulfate, activated zeolite X, activated zeolite A, activated bentonite clay, activated silica gel, and mixtures thereof.

13. A method according to claims 1-12 wherein said moisture-permeable, water-insoluble sachet is further provided in combination with a perfume composition.

14. A method according to claim 11 wherein said sachet, said perfume composition and said dehydrating agent are placed within distinct containers provided with openings so as to allow free circulation of air within said container.

15. A method according to claim 13 wherein said perfume composition is comprised within said mixture.

16. A method according to claims 1-13 wherein said perfume composition is loaded onto said sachet.

17. A method according to any of the preceding claims wherein said dry chlorine dioxide precursor and said dry chlorine dioxide activator are not capable of reacting with each other to produce chlorine dioxide gas in substantial absence of water.

18. A method according to any of the preceding claims wherein said dry chlorine dioxide precursor and said dry chlorine dioxide activator generates chlorine dioxide gas in a sustained concentration of from 0.001 ppm to 1000 ppm, preferably from 0.001 ppm to 100 ppm, more preferably from 0.01 to 10 ppm, and most preferably from 0.01 to 0.1 ppm.

19. A method according to claim 13 wherein said sachet further comprises a set of usage instructions to direct the user towards selecting, dosing and placing said sachet and/or said perfume composition and/or said dehydrating agent in a suitable manner.

20. A method according to any of the preceding claims for reducing the formation of malodors selected from the group of mold and mildew odors, odors from soap scum and body soil residues, pipe malodors, smoke, cleaning or personal care products malodors, and mixtures thereof.

21. A method according to claim 20 for reducing the formation of mold and mildew odors.

22. A method of freshening air in a bathroom, said method comprising the step of placing a moisture-permeable, water-insoluble sachet comprising a dry chlorine dioxide precursor and a dry chlorine dioxide activator in close proximity of a bathtub and/or shower present in the bathroom.

23. The use of a moisture-permeable, water-insoluble sachet comprising a dry chlorine dioxide precursor and a dry chlorine dioxide activator, for reducing the formation of malodors in a bathroom.

24. The use according to claim 23 for reducing the formation of mold and mildew odors.
